# EUROPÄISCHE PATENTSCHRIFT

(11) **EP 0 692 524 B1**
(45) Veröffentlichungstag und Bekanntmachung des Hinweises auf die Patenterteilung: **24.06.1998**
(21) Anmeldenummer: 95105417.0
(22) Anmeldetag: 11.04.1995
(51) Int. Cl.: C09C 1/30, C04B 22/06, A61K 6/02

(54) **Verfahren zur Herstellung von Kieselsäuregranulat und Verwendung des so hergestellten Granulats**
Process for preparing silica granules and their use
Procédé de préparation de granulés de silice et utilisation de ceux-ci

(30) Priorität: 11.07.1994 DE 4424044
(43) Veröffentlichungstag der Anmeldung: 17.01.1996
(73) Patentinhaber: Heraeus Quarzglas GmbH, 63450 Hanau (DE)
(72) Erfinder: Werdecker, Waltraud, D-63456 Hanau (DE); Gerhardt, Rolf, D-63546 Hammersbach (DE); Schaper, Hartwig, Dr., D-63741 Aschaffenburg (DE); Englisch, Wolfgang, Dr., D-65779 Kelkheim (DE)
(74) Vertreter: Kühn, Hans-Christian

(56) Entgegenhaltungen:
- CA-A- 1 153 507
- US-A- 4 042 361

## Beschreibung

Die Erfindung betrifft ein Verfahren zur Herstellung von Kieselsäuregranulat durch Mischen von Kieselsäurepulver mit einer Flüssigkeit.

Kieselsäurepulver sind mittels Gasphasen-Reaktionen, wie beispielsweise durch Hydrolyse von siliziumhalogeniden oder von siliziumorganischen Verbindungen, oder mittels Sol-Gel-Verfahren herstellbar. Sie fallen beispielsweise bei der Herstellung von synthetischem Quarzglas auch als Nebenprodukt in großen Mengen an. Ihre Wiederverwertung ist aber problematisch. Zwar zeichnen sich diese Kieselsäurepulver durch eine sehr hohe Reinheit aus, wegen ihrer hohen spezifischen Oberfläche und ihrer geringen Schüttdichte lassen sie sich aber nur schwer handhaben und nicht ohne weiteres mittels bekannter Verfahren zu durchsichtigen, blasenarmen Quarzglaskörpern aufschmelzen.

Um die Handhabung des feinkörnigen Kieselsäurepulvers zu erleichtern und um das Pulver für die Herstellung von Gegenständen aus Quarzglas mittels üblicher Herstellungsverfahren aufzubereiten, wird in der EP-B1 0 335 875 vorgeschlagen, die BET-Oberfläche des Kieselsäurepulvers zu verringern, indem das Kieselsäurepulver unter Ausbildung einer krümeligen Masse mit Wasser gemischt, das benetzte Pulver getrocknet und das getrocknete Pulver anschließend zerkleinert, gesiebt und getempert wird.

Ein ähnliches Verfahren ist auch aus der US-Patentschrift 4,042,361 bekannt. Bei dem dort beschriebenen Aufbereitungsverfahren für synthetisches Kieselsäurepulver wird dieses mit Wasser gemischt, das benetzte Pulver getrocknet und anschließend bei einer Temperatur zwischen 1000 °C und 1400 °C getempert. Das so hergestellte Granulat ist als Einsatzmaterial für die Herstellung von Quarzglas-Tiegeln im Schlickergieß-Verfahren geeignet.

In der Druckschrift JP 62-202827 wird zur Herstellung von Kieselsäuregranulat vorgeschlagen, Kieselsäurepulver mit einem Teilchendurchmesser von weniger als 0,1 um mit natriumhaltiger wäßriger Lösung zu mischen und zu trocknen, wobei Agglomerate zwischen 50 und 500 um entstehen. Diese Agglomerate werden anschließend gesintert und verglast.

Aus der DD-A7 291 445 ist ein Verfahren zur Herstellung eines Kieselglas-Schmelzgranulats bekannt, wobei als Ausgangsmaterial lepidoide Kieselsäure eingesetzt wird, die auf eine Korngröße unter 60 µm fein zerkleinert, anschließend bei 950 °C bis 1200 °C entwässert, dann mittels eines Siebgranulators nach Zugabe einer 1%-igen Polyvinyl-Alkohollösung in Granalien mit Korngrößen von weniger als 1 mm Durchmesser überführt wird.

In der DE-PS 34 06 185 wird für die Tablettierung von pyrogen hergestellten Siliziumdioxidpartikeln vorgeschlagen, eine Mischung aus dem pyrogen hergestelltem Oxid, Bindemittel, Wasser und Gleitmittel zu homogenisieren, durch ein Sieb zu drücken, das so erhaltene Granulat zu trocknen bis es eine für die Verpressung ausreichende Festigkeit hat, anschließend dieses Granulat zu Preßlingen zu verpressen und diese bei 600°C bis 850°C zu sintern.

Aus der EP-A1 0 578 553 ist ein Verfahren zur Herstellung von Kieselsäure-Körnung mittels einer Sol-Gel-Methode bekannt, bei der zunächst eine wäßrige Suspension aus Kieselsäure-Pulver hergestellt, diese geliert und anschließend durch Mikrowelle getrocknet und zerteilt wird. Die geeignete Kornfraktion wird danach abgesiebt.

Die bekannten Verfahren weisen eine Vielzahl von Verfahrensschritten auf, die erforderlich sind, um eine geeignete Korngrößenverteilung, eine ausreichende Verdichtung und Rieselfähigkeit des Granulate zu erzielen. Insbesondere ist auch der Trockenschritt mit einem hohen Energieaufwand verbunden.

Der vorliegenden Erfindung liegt daher die Aufgabe zugrunde, ein Verfahren für die Herstellung eines rieselfähigen Granulats mit hoher Schüttdichte und mit definierter Teilchengrößenverteilung bereitzustellen, das staubarm und gut handhabbar ist und sich als Ausgangsmaterial für Quarzglasprodukte eignet..

Diese Aufgabe wird erfindungsgemäß dadurch gelöst, daß das Mischen eine erste Mischphase, in der das Mischgut unter Bildung einer grobkörnigen Masse einer langsamen Mischbewegung unterworfen wird, und eine zweite Mischphase, in der unter Zerkleinerung und Verdichtung der grobkörnigen Masse durch eine gesteigerte Mischbewegung eine feinkörnige Masse gebildet wird, umfaßt, wobei mindestens während der zweiten Mischphase der Gehalt an Kieselsäurepulver im Mischgut auf mindestens 75 Gew.-% eingestellt wird. Das langsame Mischen ermöglicht das Herstellen einer Suspension mit einem zunächst hohen Flüssigkeitsanteil, die aufgrund ihrer rheologischen Eigenschaften ohne hohen Energieaufwand eine gute Durchmischung des Mischgutes gewährleistet und die beispielsweise nach und nach mit Kieselsäurepulver angereichert werden kann. Durch die zunächst langsame Mischbewegung während der ersten Mischphase kann leicht staubendes Kieselsäurepulver in die Flüssigkeit oder in das im Mischbehälter bereits vorhandene Mischgut schonend eingearbeitet werden.Dabei entsteht eine grobkörnige, krümeiige Masse. Durch die Intensivierung der Mischbewegung in der zweiten Mischphase wird diese grobkörnige Masse durch Zerkleinerung der Körnung und Verdichtung der einzelnen Körner zu einer feinkörnigen Masse umgebildet. Die Zerkleinerung der Körnung und die Verdichtung der neu gebildeten feineren Körnung beruhen auf einer erhöhten Schlag- und Scherbeanspruchung des Mischgutes. Diese mechanischen Kräfte wirken jedoch in diesem Sinne nur bis zu einer bestimmten endlich kleinen Korngröße. Nach einer gewissen Mischdauer erfolgt keine weitere wesentlich Zerkleinerung mehr. Die Korngröße und die Verdichtung der feinkörnigen Masse sind daher in etwa einheitlich, wobei die mittlere Korngröße und die erzielte Verdichtung im wesentlichen von der Energie der Mischbewegung in der zweiten Mischphase abhängen. Die durch die beschleunigte Mischbewegung erzeugte Verdichtung des Mischgutes trägt zu einer hohen Schüttdichte des Kieselsäuregranulats bei. Weiterhin ermöglich das Mischen in zwei definierten Mischphasen die Einstellung eines hohen Feststoffgehaltes im Mischgut. Dadurch, daß der Gehalt an Kieselsäurepulver mindestens während der zweiten Mischphase auf 75 Gew.-% oder mehr eingestellt wird, wird eine zusätzlich Verdichtung der feinkörnigen Masse erreicht.

Die so hergestellten Kieselsäuregranulate weisen bereits eine so hohe Schüttdichte bei gleichzeitig guter Rieselfähigkeit auf, daß sie leicht handhabbar und einsetzbar sind. Ein besonderer Vorteil des Verfahrens liegt darin, daß die Granulierung ohne Zusatz von Bindemitteln erfolgen kann. Derartige Bindemittel, die häufig Alkalimetalle enthalten, sind insbesondere für Hochtemperaturanwendungen der aus dem Granulat herstellbaren Quarzglasbauteile oder für Anwendungszwecke in der Halbleitertechnik schädlich. Aufgrund ihres geringen Flüssigkeitsgehaltes und ihrer hohen Dichte sind die erfindungsgemäß hergestellten Kieselsäuregranulate auch mit relativ geringem Energieaufwand zu tocknen. Sie besitzen gute Sintereigenschaften und sind mit den für Quarzglasrohstoffe üblichen Reinigungs- und Sinterverfahren zu hochwertigen Quarzglasprodukten verarbeitbar.

Aufgrund der guten Fließfähigkeit des Mischgutes während der ersten Mischphase werden dabei in das Mischgut eingebrachte Dotiermittel, wie beispielsweise pyrogen hergestelltes Aluminiumoxid, sehr schnell und gleichmäßig verteilt.

Eine besonders hohe Verdichtung wird erreicht, indem die Mischbewegung von der ersten zur zweiten Mischphase um mindestens 50 %, vorzugsweise um 100 % oder mehr gesteigert wird. Als günstig hat es sich erwiesen, wenn die Steigerung der Mischbewegung derart ist, daß beim Übergang von der ersten zur zweiten Mischphase im Inneren der Körnung vorhandene Flüssigkeit an der Oberfläche der Körner austritt. Dort läßt sich diese Flüssigkeit, sofern gewünscht, leicht abbinden. Vorteilhafterweise wird die Dauer der zweiten Mischphase so gewählt, daß sie nach dem Austreten der Flüssigkeit aus der Kieselsäurekörnung im wesentlichen beendet ist.

Bevorzugt wird die Mischbewegung in einem Mischgutbehälter mit rotierendem Wirblerwerkzeug erzeugt, dessen Umfangsgeschwindigkeit während der ersten Mischphase auf einen Wert im Bereich von 15 m/s bis 30 m/s, und in der zweiten Mischphase auf 30 m/s oder mehr eingestellt wird. Zur Erzeugung einer hohen Verdichtung und einer einheitlichen Korngrößenverteilung hat es sich als besonders günstig herausgestellt, in der zweiten Mischphase das Mischgut einer intensiven Misch- und Schlagbeanspruchung unter Verwendung eines Intensivmischers zu unterwerfen.

Kieselsäuregranulate mit besonders hoher Schüttdichte und besonders geringem Feuchtigkeitsgehalt werden erhalten, wenn der Gehalt an Kieselsäurepulver während der ersten Mischphase bereits mindestens 70 % beträgt. Dies erleichtert die nachfolgende Verdichtung und die einstellung des erforderlichen Feststoffgehaltes in der zweiten Mischphase.

Als günstig hat es sich herausgestellt, unmittelbar vor oder während der zweiten Mischphase dem Mischgut Kieselsäurepulver zuzugeben. Die schnellere Mischbewegung erlaubt eine Erhöhung der Viskosjtät des Mischgutes während der zweiten Mischphase, ohne daß die Durchmischung des Mischgutes und die Verdichtung der Körnung dadurch wesentlich erschwert würden.

Bevorzugt wird eine Verfahrensweise, bei der das Mischen eine dritte Mischphase umfaßt, vor oder während der dem Mischgut Kieselsäurepulver zugegeben wird, wobei beim Übergang von der zweiten auf die dritte Mischphase die Mischbewegung nicht wesentlich geändert wird. Dadurch bleibt der vorher eingestellte Verdichtungsgrad des Mischgutes und die vorgegebene mittlere Körnungsgröße erhalten. In den vorangehenden Mischphasen an die Oberfläche der Körner ausgetretene Feuchtigkeit kann durch die Zugabe von weiterem Kieselsäurepulver abgepudert werden. Durch das "Abpudern" wird die Agglomeration des feuchten Granulats verhindert und dessen Rieselfähigkeit verbessert. Gleichzeitig wird durch die Zugabe von Kieselsäurepulver eine weitere Erhöhung des Feststoffgehaltes im Mischgut erreicht.

Es hat sich als günstig erwiesen, die mittlere Korngröße der grobkörnigen Masse auf einen Wert im Bereich von 1 mm bis 4 mm und die der feinkörnigen Masse auf einen Wert von weniger als 1 mm, vorzugsweise auf 90 µm bis 350 µm, einzustellen. Die Einstellung der Korngröße erfolgt über den Flüssigkeitsgehalt des Mischgutes und, insbesondere in der zweiten Mischphase über die Intensität der Mischbewegung.

Um fließfähige Kieselsäuregranulate mit hoher Schüttdichte zu erzeugen hat sich als Ausgangsmaterial amorpher Kieselsäurestaub mit einer mittleren Korngröße von weniger als 1 µm und mit einer BET-Oberfläche von mehr als 40 m²/g als besonders geeignet erwiesen.

Vorteilhafterweise wird zur Erzielung einer hohen chemischen Reinheit des Kieselsäuregranulats entmineralisiertes Wasser mit einer elektrischen Leitfähigkeit von weniger als 1 µS eingesetzt.

Überraschend hat sich das erfindungsgemäße Kieselsäuregranulat als besonders gut geeignetes Ausgangsmaterial für die Herstellung von anorganischen Füllstoffen, zum Beispiel in Dentalmaterialien erwiesen. Derartige Materialien werden beispielsweise als feinteilige Füllstoffe in organischen Kunststoffen in der Dentaltechnik eingesetzt. Das erfindungsgemäße Kieselsäuregranulat ist aufgrund seiner Größe und seiner hohen Verdichtung (um ca. das 10-fache des Ausgangs-Kieselsäurepulvers) staubarm und gut handhabbar. Es läßt sich beim Einarbeiten leicht verreiben und gleichmäßig verteilen. Zur Entfernung der Restfeuchte kann es bei Temperaturen zwischen 80 °C und 600 °C getrocknet werden, ohne daß es sich in seiner Struktur und Rieselfähikeit verändert. Um eine leichte Verfestigung zu erreichen, wie sie beispielsweise für die Anwendung als Füllstoff im Dentalbereich sinnvoll ist, ist eine Temperaturbehandlung um 900 °C vorteilhaft. Bei einer entsprechenden Anpassung der Korngrößenverteilung ist das Granulat aber auch für den Einsatz als Katalysatorträger mit hoher Abriebfestigkeit geeignet. Hierfür hat sich eine Temperaturbehandlung des Granulats im Bereich zwischen 1000 °C und 1200 °C als günstig herausgestellt.

Das erfindungsgemäße Verfahren wird nachfolgend anhand eines Ausführungsbeispiels näher erläutert.

Durch Mischen von 14 kg eines amorphen Kieselsäurestaubes mit Teilchengrößen von 10 nm bis 100 nm und einer spezifischen Oberfläche von etwa 70 m²/g mit 17 kg entmineralisiertem Wasser, das eine elektrische Leitfähigkeit von 0,1 µS aufweist, wird in einem Schnell-Mischer mit azentrisch ausgebrachtem Stiftwerkzeug eine wäßrige Suspension hergestellt. Der Schnell-mischer wird dabei mit einer Umdrehungsgeschwindigkeit von 750 U/min (das entspricht einer Umfangsgeschwindigkeit von 15,7 m/s) betrieben. In die Suspension werden weitere 28 kg amorphen Kieselsäurestaubes eingerührt. Nach einer Mischdauer von ca. 3 Minuten liegt das Mischgut als körnige Masse mit einem mittleren Korndurchmesser von weniger als 4 mm vor.

In einer zweiten Mischphase werden dem Mischgut weitere 11 kg amorpher Kieselsäurestaub zugegeben und die Umdrehungsgeschwindigkeit des Stiftwerkzeuges auf 1500 U/min (entspricht einer Umdrehungsgeschwindigkeit von 31,4 m/s) verdoppelt. Dabei findet eine Schlag- und Scherbeanspruchung der körnigen Masse statt, die zu einer Zerkleinerung und Verdichtung der Körnung führt. Gleichzeitig wird ein Teil des Wassers an die Oberfläche der Körnung ausgepreßt. Das Austreten des Wassers an die Oberfläche ist nach ca. 20 Minuten beendet. Um ein Verkleben der so erzeugten feinkörnigen Masse durch das ausgepreßte Wasser zu verhindern, werden bei weiterhin mit einer Umdrehungsgeschwindigkeit von 1500 U/min rotierendem Stiftwerkzeug weitere 2 kg Kieselsäurestaub dem Mischgut hinzugegeben um damit die Oberfläche der Körnung innerhalb einer Mischdauer von ca. 15 Sekunden gleichmäßig abzupudern.

Das so erzeugte feinkörnige Granulat ist fließfähig, bindemittelfrei und hat eine definierte Teilchengrößenverteilung. Diese liegt im Bereich von 90 µm bis 350 µm. Es weist eine hohe Festigkeit auf und ist daher leicht handhabbar. Es hat eine Restfeuchte von weniger als 24 Gew.-%. Nach Entfernender Restfeuchte und gegebenenfalls einer Teilverfestigung bei niedrigen Tempearturen ist es unmittelbar als "temporäres" Granulat für die Herstellung von anorganischen Füllstoffen, etwa in Dentalmaterialien oder als sinteraktiver Syntheserohstoff, beispielsweise für die Mullitherstellung, einsetzbar. Hierzu wird das Granulat unmittelbar beim Einarbeiten zerrieben.

Durch eine thermische Behandlung bei Temperaturen zwischen 1000 °C bis 1200 °C läßt sich das Granulat weiter verfestigen, ohne daß die spezifische Oberfläche wesentlich abnimmt. Ein derartiges Granulat, das ein großes Porenvolumen aufweist, ist beispielsweise für die Herstellung von Katalysatorträgern gut geeignet.

Durch eine Temperaturbehandlung bei Temperaturen oberhalb von ca. 1350 °C läßt sich die spezifische Oberfläche des Granulats auf Werte von weniger als 1 m²/g verringern. Ein derartiges Granulat ohne offene Poren ist als Einsatzmaterial für die Herstellung von Quarzglas geeignet.

## Patentansprüche

1. Verfahren zur Herstellung von Kieselsäuregranulat durch Mischen von Kieselsäurepulver mit einer Flüssigkeit, wobei das Mischen eine erste Mischphase, in der das Mischgut unter Bildung einer grobkörnigen Masse einer langsamen Mischbewegung unterworfen wird, und eine zweite Mischphase, in der unter Zerkleinerung und Verdichtung der grobkörnigen Masse durch eine gesteigerte Mischbewegung eine feinkörnige Masse gebildet wird, umfaßt, dadurch gekennzeichnet, daß mindestens während der zweiten Mischphase der Gehalt an Kieselsäurepulver im Mischgut auf mindestens 75 Gew.-% eingestellt wird.

2. Verfahren nach Anspruch 1, dadurch gekennzeichnet, daß die Mischbewegung von der ersten zur zweiten Mischphase um mindestens 50 %, vorzugsweise um 100 % oder mehr gesteigert wird.

3. Verfahren nach Anspruch 1 oder 2, dadurch gekennzeichnet, daß die Mischbewegung beim Übergang von der ersten zur zweiten Mischphase derart gesteigert wird, daß im Inneren der Körnung vorhandene Flüssigkeit an die Oberfläche der Körner tritt, wobei die Dauer der zweiten Mischphase so gewählt wird, daß der Austritt der Flüssigkeit im wesentlichen beendet ist.

4. Verfahren nach einem der vorhergehenden Ansprüche, dadurch gekennzeichnet, daß die Mischbewegung in einem Mischgutbehälter mit rotierendem Wirblerwerkzeug erzeugt wird, dessen Umfangsgeschwindigkeit während der ersten Mischphase auf einen Wert im Bereich von 15 m/s bis 30 m/s, und in der zweiten Mischphase auf 30 m/s oder mehr eingestellt wird.

5. Verfahren nach einem der vorhergehenden Ansprüche, dadurch gekennzeichnet, daß in der zweiten Mischphase das Mischgut einer intensiven Misch- und Schlagbeanspruchung unter Verwendung eines Intensivmischers unterworfen wird.

6. Verfahren nach einem der vorhergehenden Ansprüche, dadurch gekennzeichnet, daß der Gehalt an Kieselsäurepulver während der ersten Mischphase mindestens 70 % beträgt.

7. Verfahren nach einem der vorhergehenden Ansprüche, dadurch gekennzeichnet, daß unmittelbar vor oder während der zweiten Mischphase dem Mischgut Kieselsäurepulver zugegeben wird.

8. Verfahren nach einem der vorhergehenden Ansprüche, dadurch gekennzeichnet, daß das Mischen eine dritte Mischphase umfaßt, vor oder während der dem Mischgut Kieselsäurepulver zugegeben wird, wobei beim Übergang von der zweiten auf die dritte Mischphase die Mischbewegung nicht wesentlich geändert wird.

9. Verfahren nach einem der vorhergehenden Ansprüche, dadurch gekennzeichnet,daß die mittlere Korngröße der grobkörnigen Masse auf einen Wert im Bereich von 1 mm bis 4 mm und die der feinkörnigen Masse auf einen Wert von weniger als 1 mm, vorzugsweise von 90 µm bis 350 µm, eingestellt wird.

10. Verfahren nach einem der vorhergehenden Ansprüche, dadurch gekennzeichnet, daß als Kieselsäurepulver amorpher Kieselsäurestaub mit einer mittleren Korngröße von weniger als 1 µm und mit einer BET-Oberfläche von mehr als 40 m²/g eingesetzt wird.

11. Verfahren nach einem der vorhergehenden Ansprüche, dadurch gekennzeichnet, daß als Flüssigkeit Wasser mit einer elektrischen Leitfähigkeit von weniger als 1 µS eingesetzt wird.

12. Verwendung eines nach den Ansprüchen 1 bis 11 hergestellten Kieselsäuregranulats als Ausgangsmaterial für die Herstellung von anorganischen Füllstoffen in Dentalmaterialien.

13. Verwendung nach Anspruch 12, dadurch gekennzeichnet, daß das Granulats mittels einer Temperaturbehandlung im Bereich um 900 °C verfestigt wird.

## Claims

1. A method for the production of silica granulate by the mixing of silica powder with a fluid, in which the mixing comprises a first mixing phase, in which the mixing material is subjected to a slow mixing movement with the formation of a coarse-grained material, and a second mixing phase, in which a fine-grained material is formed with comminution and compacting of the coarse-grained material by an increased mixing movement, characterised in that at least during the second mixing phase the content of silica powder in the mixing material is set at at least 75 % by weight.

2. The method according to Claim 1, characterised in that the mixing movement is increased from the first to the second mixing phase by at least 50 %, preferably by 100 % or more.

3. The method according to Claim 1 or 2, characterised in that the mixing movement on transition from the first to the second mixing phase is increased such that fluid present inside the granulation strikes against the surface of the grains, in which the duration of the second mixing phase is selected so that the emergence of the fluid is substantially terminated.

4. The method according to one of the preceding claims, characterised in that the mixing movement is provided in a mixing material vessel with rotating centrifugal tool, the circumferential speed of which during the first mixing phase is set at a value in the range of 15 m/s to 30 m/s and in the second mixing phase to 30 m/s or more.

5. The method according to one of the preceding claims, characterised in that in the second mixing phase, the mixing material is subjected to an intensive mixing- and impact stress with the use of an intensive mixer.

6. The method according to one of the preceding claims, characterised in that the content of silica powder during the first mixing phase amounts to at least 70 %.

7. The method according to one of the preceding claims, characterised in that immediately before or during the second mixing phase, silica powder is added to the mixing material.

8. The method according to one of the preceding claims, characterised in that the mixing comprises a third mixing phase, before or during which silica powder is added to the mixing material, in which on transition from the second to the third mixing phase, the mixing movement is not substantially altered.

9. The method according to one of the preceding claims, characterised in that the mean grain size of the coarse-grained material is set at a value in the range of 1 mm to 4 mm and that of the fine-grained material to a value of less than 1 mm, preferably of 90 µm to 350 µm.

10. The method according to one of the preceding claims, characterised in that as silica powder, amorphous silica dust is used with a mean grain size of less than 1 µm and with a BET surface of more than 40 m²/g.

11. The method according to one of the preceding claims, characterised in that as fluid, water is used with an electrical conductivity of less than 1 µS.

12. The use of a silica granulate, produced according to Claims 1 to 11, as initial material for the production of inorganic fillers in dental materials.

13. The use according to Claim 12, characterised in that the granulate is solidified by means of a temperature treatment in the range of around 900° C.

## Revendications

1. Procédé de préparation de granulés de silice par mélange de poudre de silice avec un liquide, selon lequel le mélange comprend une première phase de mélange dans laquelle le matériau de mélange est soumis à un mouvement de mélange lent avec formation d'une masse à gros grains, et une deuxième phase de mélange dans laquelle est formée une masse à grains fins par fragmentation et compression de la masse en gros grains par un mouvement de mélange augmenté, caractérisé en ce qu'au moins pendant la deuxième phase de mélange la teneur en poudre de silice dans le matériau de mélange est réglée à au moins 75 % en poids.

2. Procédé selon la revendication 1, caractérisé en ce que le mouvement de mélange de la première à la deuxième phase de mélange est augmenté d'au moins 50 %, de préférence 100 % ou plus.

3. Procédé selon la revendication 1 ou 2, caractérisé en ce que le mouvement de mélange en passant de la première à la deuxième phase de mélange est augmenté de manière que le liquide existant à l'intérieur du grain se rassemble à la surface du grain, la durée de la deuxième phase étant choisie de manière que le rassemblement du liquide soit substantiellement complet.

4. Procédé selon l'une des revendications précédentes, caractérisé en ce que le mouvement de mélange dans un conteneur de matériau de mélange est réalisé avec un outil à tourbillon rotatif dont la vitesse circonférentielle pendant la première phase de mélange est réglée à une valeur dans le domaine de 15 m/s à 30 m/s et pendant la deuxième phase de mélange de 30 m/s ou plus.

5. Procédé selon l'une des revendications précédentes, caractérisé en ce que dans la deuxième phase de mélange le matériau de mélange est soumis à une contrainte de mélange et de choc intensive par l'utilisation d'un mélangeur intensif.

6. Procédé selon l'une des revendications précédentes, caractérisé en ce que la teneur en poudre de silice pendant la première phase de mélange est d'au moins 70 %.

7. Procédé selon l'une des revendications précédentes, caractérisé en ce que directement avant ou pendant la deuxième phase de mélange on ajoute de la poudre de silice au matériau de mélange.

8. Procédé selon l'une des revendications précédentes, caractérisé en ce que le mélange comprend une troisième phase de mélange, avant ou après laquelle on ajoute de la poudre de silice au matériau de mélange, le mouvement du mélange pour le passage de la deuxième à la troisième phase de mélange n'est pas sensiblement modifié.

9. Procédé selon l'une des revendications précédentes, caractérisé en ce que la granulométrie moyenne de la masse à gros grains est réglée dans un domaine de 1 mm à 4 mm et celle de la masse à grains fins est réglée à une valeur de moins que 1 mm, de préférence de 90 µm à 350 µm.

10. Procédé selon l'une des revendications précédentes, caractérisé en ce qu'on utilise comme poudre de silice une poussière de silice amorphe avec une granulométrie moyenne de moins que 1 µm et avec une surface BET de plus que 40 m²/g.

11. Procédé selon l'une des revendications précédentes, caractérisé en ce qu'on utilise comme liquide de l'eau avec une conductibilité électrique de moins que 1 µS.

12. Utilisation de granulés de silice fabriqué selon l'une des revendications 1 à 11 comme matériau de départ pour la préparation de charges minérales dans les matériaux dentaires.

13. Utilisation selon la revendication 12, caractérisée en ce qu'on consolide les granulés au moyen d'un traitement thermique dans le domaine de 900 °C.
